# FASCICULE DE BREVET EUROPEEN

(11) **EP 3 234 102 B1**
(45) Date de publication et mention de la délivrance du brevet: **27.06.2018**
(21) Numéro de dépôt: 15831060.7
(22) Date de dépôt: 17.12.2015
(51) Int. Cl.: C12M 1/26, B29C 67/00, B41J 2/00, C12M 3/00, C12N 5/071, C12M 1/00

(54) **PROCÉDÉ D'IMPRESSION D'ÉLÉMENTS BIOLOGIQUES PAR LASER ET DISPOSITIF POUR SA MISE EN OEUVRE**
VERFAHREN ZUM LASERDRUCKEN VON BIOLOGISCHEN KOMPONENTEN UND VORRICHTUNG ZUR IMPLEMENTIERUNG DES BESAGTEN VERFAHRENS
METHOD FOR LASER PRINTING BIOLOGICAL COMPONENTS, AND DEVICE FOR IMPLEMENTING SAID METHOD

(30) Priorité: 17.12.2014 FR 1462570
(43) Date de publication de la demande: 25.10.2017
(73) Titulaire: Université de Bordeaux, 33000 Bordeaux (FR); INSERM - Institut National de la Santé et de la Recherche Médicale, 75013 Paris (FR)
(72) Inventeur: GUILLEMOT, Fabien, 33210 Preignac (FR)
(74) Mandataire: IP Trust
(86) Numéro de dépôt international: PCT/FR2015/053570
(87) Numéro de publication internationale: WO 2016/097620

(56) Documents cités:
- WO-A1-2014/110590
- US-A1- 2013 017 564
- SAUNDERS ET AL.: "Delivery of human fibroblast cells by piezoelectric drop-on-demand inkjet printing", BIOMATERIALS, vol. 29, no. 2, 23 octobre 2007 (2007-10-23), pages 193-203, XP022323370,
- OVSIANIKOV ET AL.: "Laser printing of cells into 3D scaffolds", BIOFABRICATION, vol. 2, no. 1, 10 mars 2010 (2010-03-10), XP002665511,

## Description

La présente invention se rapporte à un procédé d'impression d'éléments biologiques par laser ainsi qu'à un dispositif pour sa mise en oeuvre. L'invention a également pour objet un procédé de réalisation d'un tissu biologique qui utilise ce procédé d'impression.

Selon un premier mode opératoire, il est possible de faire croître in vitro des cellules sur une matrice macroporeuse biodégradable (scaffold en anglais) pour obtenir un tissu biologique. Ce premier mode opératoire n'est pas pleinement satisfaisant car la croissance du tissu biologique peut être limitée dans la profondeur de la matrice macroporeuse en raison d'un défaut de colonisation cellulaire. En outre, ce mode opératoire permet difficilement de traiter de la complexité des tissus (qui se caractérise par une multiplicité de types cellulaires organisés selon des arrangements spécifiques et généralement anisotropes).

Pour remédier à ces inconvénients, des procédés d'impression d'éléments biologiques ont été développés pour produire un tissu biologique de manière automatisable.

Selon les ouvrages, ces procédés d'impression sont appelés bio-impression, microimpression d'éléments biologiques ou bioprinting en anglais.

Selon ces procédés, le tissu biologique est obtenu en imprimant des gouttelettes d'encres biologiques. Pour obtenir un volume, les gouttelettes sont agencées par couche qui sont superposées les unes sur les autres.

Selon une première variante, l'encre biologique est stockée dans un réservoir et passe à travers des buses ou des capillaires pour former des gouttelettes qui sont transférées sur un support. Cette première variante d'impression dite à buse regroupe la bioextrusion, l'impression jet d'encre ou l'impression par microvannes.

La bioextrusion permet d'obtenir une densité cellulaire importante de l'ordre de 100 millions de cellules par millilitre et une résolution de l'ordre du millimètre.

L'impression par microvannes permet d'obtenir une densité cellulaire moindre de l'ordre de quelques millions de cellules par millilitre et une résolution meilleure de l'ordre de 100 µ 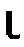 η. L'impression jet d'encre permet d'obtenir une densité cellulaire identique à l'impression par microvannes, inférieure à 10 millions de cellules par millilitre et une résolution meilleure de l'ordre de 10 µ 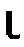 η).

Dans le cas de la bioextrusion, les cellules sont déposées à partir d'une première buse et un hydrogel est simultanément déposé à partir d'une seconde buse. En variante, les cellules et l'hydrogel sont mixés dans un réservoir avant extrusion. Dans les deux autres cas, l'encre est un milieu aqueux contenant les cellules. Selon les variantes, la bioextrusion permet de déposer l'encre de manière continue sous la forme de filaments ou de manière discontinue sous forme de gouttelettes.

Selon ces modes d'impression à buse, la résolution d'impression étant liée notamment à la section des buses, seules des encres biologiques avec des caractéristiques rhéologiques données peuvent être utilisées pour des résolutions élevées. Ainsi, des encres biologiques à forte densité cellulaire peuvent être difficilement imprimées avec une résolution élevée car cette technique d'impression induit au moment du passage à travers la buse des contraintes de cisaillement importantes susceptibles d'endommager les cellules. De plus, avec ce type d'encre, les risques d'obturation des buses par les cellules sont importants du fait notamment de la sédimentation des cellules à l'intérieur des réservoirs.

Pour pouvoir utiliser une large gamme d'encres biologiques et atteindre un niveau élevé de résolution, un procédé d'impression d'éléments biologiques par laser a été développé. Ce procédé d'impression appelé bio-impression par laser, est également connu sous le nom de « Laser-Assisted Bioprinting » (LAB) en anglais. L'invention se rapporte plus précisément à ce type de procédés d'impression. A titre de comparaison, la bio-impression par laser permet d'imprimer des encres présentant une forte densité cellulaire de l'ordre de 100 millions de cellules par millilitre avec une résolution de 10 µ 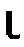 η.

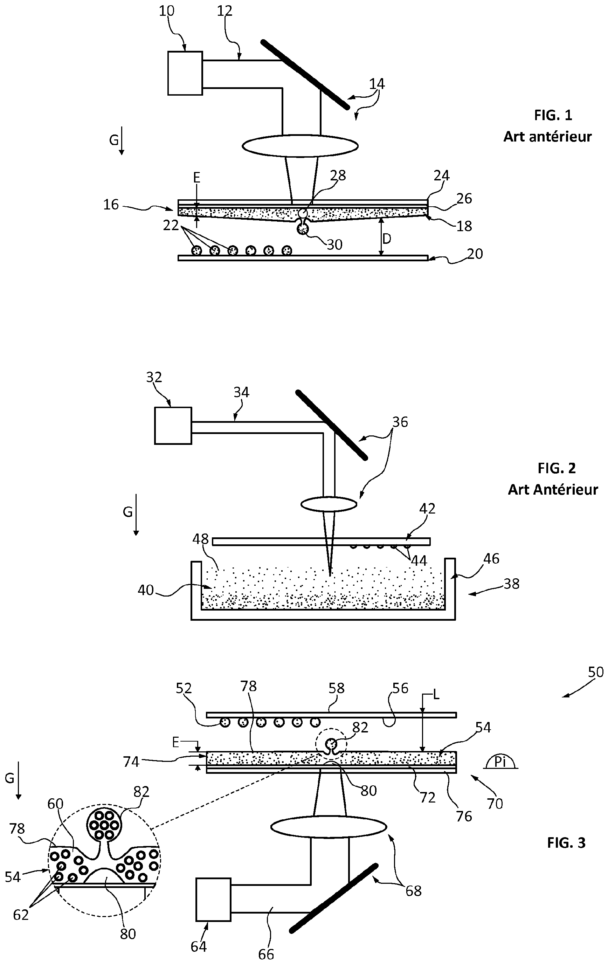

Comme illustré sur la figure 1, un dispositif d'impression d'éléments biologiques par laser qui repose sur la technique dénommée « Laser-Induced Forward Transfer » (LIFT) en anglais, comprend une source laser puisée 10 émettant un faisceau laser 12, un système 14 pour focaliser et orienter le faisceau laser 12, un support donneur 16 qui comporte au moins une encre biologique 18 et un substrat receveur 20 positionné de manière à recevoir des gouttelettes 22 émises depuis le support donneur 16.

On connaît notamment les documents de l'art antérieur suivant :
- la demande de brevet américain US2013017564
- la demande de brevet internationale WO2014110590
- l'article Saunders et al. « 3. Delivery of human fibroblast cells by piezoelectric drop-on-demand inkjet printing» paru dans BIOMATERIALS, 20071023 ELSEVIER SCIENCE PUBLISHERS BV., BARKING, GB, Vol:29,Nr:2,Page(s):193 - 203
- l'article Ovisanikov « Laser printing of cells into 3D scaffolds » paru dans BIOFABRICATION, 20100310 Institute of Physics Publishing Ltd, UK, Vol:2,Nr:l,Page(s):14104-1 - 14104-7

Selon cette technique d'impression, le faisceau laser est puisé et à chaque impulsion une gouttelette est générée. L'encre biologique 18 comprend une matrice, par exemple un milieu aqueux, dans laquelle sont présents des éléments, par exemple des cellules, à déposer sur le substrat receveur 20. Le support donneur 16 comprend une lame 24 transparente à la longueur d'onde du faisceau laser 12 qui est revêtue d'une couche absorbante 26 sur laquelle est apposée l'encre biologique 18 sous la forme d'un film.

La couche absorbante 26 permet de convertir l'énergie lumineuse en énergie cinétique. Ainsi, le faisceau laser 12 produit un échauffement ponctuel au niveau de la couche absorbante 26 qui génère par vaporisation une bulle de gaz 28 qui par expansion provoque l'éjection d'une gouttelette 30 d'encre biologique.

Selon un agencement connu, le faisceau laser 12 impacte le support donneur 16 en étant orienté selon une direction approximativement verticale et selon un sens de haut en bas, soit dans le même sens que la force gravitationnelle G. Ainsi, l'encre biologique 18 est placée sous la lame 24 de manière à être orientée vers le bas en direction du substrat receveur 20 qui est placé sous le support donneur 16.

Compte tenu de cet agencement, l'encre biologique 18 est sous la forme d'un film avec une épaisseur E inférieure à un seuil donné pour pouvoir être maintenue sur la lame. Ce seuil varie notamment en fonction de la tension de surface, de la viscosité et de la densité de l'encre biologique.

La formation des gouttelettes 30 à partir du film d'encre biologique dépend de nombreux paramètres qui sont relatifs notamment au faisceau laser 12 (longueur d'onde, énergie, durée d'impulsion,...), à la nature de l'encre biologique 18 (tension de surface, viscosité,...), aux conditions extérieures (température, hygrométrie,...).

Même si l'impression laser permet d'obtenir en théorie un débit d'impression important dans la mesure où il est possible de générer plusieurs dizaines de milliers de gouttelettes par seconde, la production de tissus biologiques n'est pas rapide car ces gouttelettes ne font que quelques dizaines de picolitres. De plus, les supports donneurs doivent être remplacés fréquemment car ils ne contiennent chacun qu'un faible volume d'encre à imprimer de l'ordre de quelques dizaines de microlitres.

Selon un deuxième mode de réalisation illustré sur la figure 2 et décrit dans une publication intitulée « Microdroplet déposition through a film-free laser forward technique » parue le 1 Octobre 2011 sur le site www.elsevier.com, un dispositif d'impression laser comprend une source laser 32 émettant un faisceau laser 34, un système 36 pour focaliser et orienter le faisceau laser 34, un support donneur 38 qui contient au moins une encre biologique 40 ainsi qu'un substrat receveur 42 positionné de manière à recevoir des gouttelettes 44 émises depuis le support donneur 38.

Contrairement au premier mode de réalisation, le support donneur 38 ne comprend pas de couche absorbante distincte de l'encre biologique.

Selon ce mode de réalisation, le support donneur 38 comprend un réservoir 46 sans paroi supérieure de sorte que la surface libre 48 de l'encre biologique 40 contenue dans le réservoir fait face au substrat receveur 42. Pour obtenir une surface libre 48 régulière et sensiblement plane, l'encre biologique ne se présente pas sous la forme d'un film mince mais d'un volume ayant une profondeur de l'ordre de 3 mm. De la sorte, le fond du réservoir n'a pas d'influence sur la forme de la surface libre 48 de l'encre biologique et les parois latérales du réservoir ont un effet limité à la périphérie de la surface libre 48 en raison de la tension de surface.

Compte tenu de la profondeur du volume d'encre biologique, la surface libre 48 est nécessairement orientée vers le haut pour rester dans le réservoir et le substrat receveur 42 est positionné au-dessus de l'encre biologique 40.

Selon ce document, pour obtenir l'éjection d'une gouttelette, le faisceau laser 34 est focalisé juste en-dessous de la surface libre 48 a une profondeur de l'ordre de 40 à 80 µ 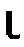 η. Ainsi, les gouttelettes émises à partir de la surface libre 48 sont projetées vers le substrat receveur 42 selon un sens de déplacement contraire au sens de la force gravitationnelle G.

Même si ce deuxième mode de réalisation utilise des supports donneurs contenant un volume d'encre biologique plus important, il n'est pas forcément adapté aux encres biologiques. En effet, comme indiqué précédemment, ces encres biologiques contiennent des éléments à imprimer, comme par exemple des cellules, noyés dans une matrice, qui ont tendance par sédimentation à descendre en fond de réservoir. La concentration en éléments à imprimer étant faible à proximité de la surface libre, les gouttelettes imprimées ont de fait de faibles concentrations en cellules ce qui est généralement préjudiciable pour le tissu biologique imprimé. De plus, selon cette méthode, il est très difficile de contrôler le nombre de cellules et la concentration des cellules déposées.

Aussi, la présente invention vise à remédier aux inconvénients de l'art antérieur.

A cet effet, l'invention a pour objet un procédé d'impression d'au moins une encre biologique, ledit procédé utilisant au moins une tête d'impression de type laser pour déposer au moins une gouttelette d'au moins une encre biologique sur une surface de dépose d'un substrat receveur. Le procédé d'impression se caractérise en ce qu'il utilise au moins une tête d'impression à buse pour déposer au moins une gouttelette d'au moins une encre biologique sur une surface de dépose du même substrat receveur que la tête d'impression de type laser.

Cette combinaison permet d'augmenter le débit de fabrication des tissus biologiques et d'obtenir des tissus biologiques plus complexes.

Pour la production d'un tissu biologique comprenant à la fois des cellules et une matrice extracellulaire, les matériaux constituant cette matrice extracellulaire sont déposés par la ou les tête(s) d'impression à buse et en ce que les cellules sont déposées par la ou les tête(s) d'impression de type laser.

L'invention a également pour objet un procédé de réalisation d'un tissu biologique qui se caractérise en ce qu'il comprend les étapes visant à :
- Générer une représentation numérique en trois dimensions du tissu biologique à réaliser, ladite représentation comprenant plusieurs zones volumiques colorées ou texturées, chaque couleur ou texture étant associée à une encre biologique,
- Trancher la représentation numérique en une succession de couches superposées, chaque couche comprenant des zones colorées ou texturées qui correspondent aux zones volumiques de la représentation numérique,
- Pour chaque couche, déterminer la position des gouttelettes à imprimer de chaque encre biologique en fonction des zones colorées ou texturées et du volume prévu de chacune des gouttelettes,
- Imprimer les différentes gouttelettes.

De préférence, les couches ont une épaisseur fonction des dimensions des gouttelettes. Avantageusement, chaque couche comprend un ensemble de petits polygones élémentaires qui ont des couleurs ou des textures différentes en fonction de la zone à laquelle ils appartiennent.

Selon un mode opératoire, pour déterminer la position de chaque gouttelette, chaque zone d'une même couleur ou d'une même texture est remplie par des ellipses identiques qui ont des dimensions fonction des dimensions des gouttelettes de l'encre biologique destinée à être imprimée dans ladite zone, le centre de chaque ellipse correspondant à la position du centre d'une gouttelette. De préférence, le positionnement des ellipses est réalisé zone par zone, par ordre décroissant de grosseurs.

L'invention a également pour objet un dispositif d'impression qui comprend au moins un substrat receveur avec une surface de dépose et au moins une tête d'impression de type laser et qui se caractérise en ce qu'il comprend au moins une tête d'impression à buse pour imprimer au moins une encre biologique sur le même substrat receveur que la ou les tête(s) d'impression de type laser.

Selon une autre caractéristique, le dispositif d'impression comprend une enceinte configurée pour stocker au moins un socle supportant un support donneur, ladite enceinte étant équipée de moyens de confinement permettant de conserver à l'intérieur une atmosphère adaptée aux encres biologiques.

De préférence, l'enceinte a des dimensions adaptées pour stocker plusieurs socles. Dans ce cas, le dispositif d'impression comprend au moins une embase configurée pour être stockée à l'intérieur de ladite enceinte, ladite embase comprenant un logement pour chaque socle. Avantageusement, l'enceinte comprend sur une première face orientée vers les têtes d'impression une première ouverture permettant de faire sortir les socles et sur une autre face une deuxième ouverture permettant d'introduire les socles.

Selon une autre caractéristique, le dispositif d'impression comprend une pince mobile pour déplacer les socles entre l'enceinte et la tête d'impression de type laser.

Selon une autre caractéristique, le dispositif d'impression comprend un châssis mobile supportant au moins un substrat récepteur, un système de guidage et de déplacement du châssis mobile par rapport à un bâti selon trois directions et un système de commande permettant de contrôler les déplacements du châssis mobile, ledit système de guidage et de déplacement et ledit système de contrôle ayant une précision micrométrique.

D'autres caractéristiques et avantages ressortiront de la description qui va suivre de l'invention, description donnée à titre d'exemple uniquement, en regard des dessins annexés sur lesquels :
- La figure 1 est une représentation schématique d'un dispositif d'impression par laser qui illustre une variante de l'art antérieur,
- La figure 2 est une représentation schématique d'un dispositif d'impression par laser qui illustre une autre variante de l'invention, La figure 3 est une représentation schématique d'un dispositif d'impression par laser qui illustre l'invention,
- Les figures 4A à 4D sont des vues de côté illustrant la formation ou non d'une gouttelette en fonction de différents régimes,
- Les figures 5A à 5D sont des schémas qui illustrent une gouttelette à différents moments de sa formation, la dernière figure 5D illustrant l'instant où une gouttelette atteint un substrat receveur,
- La figure 6 est une coupe d'un support donneur illustrant le rapport entre la taille des éléments à imprimer et l'épaisseur d'un film d'encre biologique,
- Les figures 7A et 7B sont des vues de côté illustrant la formation d'une excroissance à la surface libre d'un film d'encre biologique, préalablement à la formation d'une gouttelette, au même instant mais produite avec des énergies différentes pour le faisceau laser,
- La figure 8 est une représentation schématique d'un dispositif d'impression selon un mode de réalisation de l'invention combinant au moins une tête d'impression de type laser et au moins une tête d'impression de type jet d'encre,
- La figure 9 est une vue en perspective d'un dispositif d'impression selon un mode de réalisation de l'invention combinant une tête d'impression de type laser et plusieurs têtes d'impression de type jet d'encre,
- La figure 10 est une vue en perspective d'une partie du dispositif d'impression de la figure 9 lors d'une impression avec une des têtes d'impression de type jet d'encre,
- La figure 11 est une coupe d'une partie du dispositif d'impression de la figure 9 lors d'une impression avec la tête d'impression de type laser,
- La figure 12 est une vue en perspectif d'une représentation en 3 dimensions d'une partie d'un tissu biologique que l'on cherche à reproduire,
- La figure 13 est une vue en perspective d'une tranche de la représentation de la figure 12,
- La figure 14 est une vue de dessus de la tranche de la figure 13 illustrant le positionnement des gouttelettes d'encres biologiques.

Sur la figure 3, on a représenté un dispositif d'impression 50 pour réaliser au moins un tissu biologique en assemblant couche par couche, selon un agencement prédéfini, différents constituants comme par exemple une matrice extracellulaire et différents morphogènes. Ainsi, le dispositif d'impression 50 permet de déposer couche par couche des gouttelettes 52 d'au moins une encre biologique 54 sur une surface de dépose 56 qui correspond à la surface d'un substrat receveur 58 pour la première couche ou à la dernière couche déposée sur ledit substrat receveur 58 pour les couches suivantes.

Dans un but de simplifier la représentation, la surface de dépose 56 correspond à la surface du substrat receveur 58 sur la figure 3.

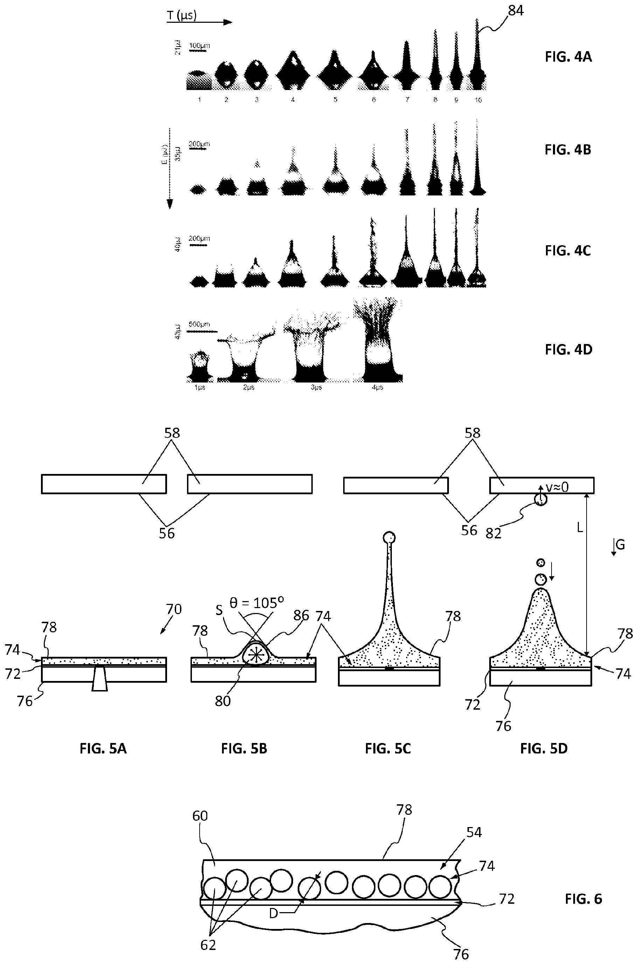

Selon un mode de réalisation sur la figure 6, l'encre biologique 54 comprend une matrice 60, par exemple un milieu aqueux, dans laquelle sont présents des éléments 62, par exemple des cellules ou des agrégats de cellules, à imprimer sur la surface de dépose 56.

Selon les cas, une encre biologique 54 comprend dans la matrice 60 une seule sorte d'éléments à imprimer 62 ou plusieurs sortes d'éléments à imprimer 62. En variante, l'encre biologique 54 peut ne comprendre qu'un seul constituant.

Par encre biologique, on entend pour la présente demande de brevet une matière biologique ou biomatériau. A titre d'exemple, l'encre biologique comprend seulement une matrice extracellulaire (par exemple du collagène), une matrice extracellulaire et des éléments tels que des cellules ou des agrégats de cellules, un milieu aqueux contenant des éléments tels que des cellules ou des agrégats de cellules.

L'encre biologique 54 n'est pas plus décrite car elle peut avoir différentes natures et des caractéristiques rhéologiques différentes d'une encre à l'autre.

Ce dispositif d'impression comprend une source laser 64 configurée pour émettre un faisceau laser 66 qui se caractérise entre autre par sa longueur d'onde, sa fréquence, son énergie, son diamètre, sa durée d'impulsion. De préférence, la source laser 64 est paramétrable afin d'ajuster au moins une caractéristique du faisceau laser, notamment son énergie.

Pour pouvoir former des gouttelettes dissociées les unes des autres, la source laser 64 est une source puisée. Pour donner un ordre de grandeur, il est possible d'éjecter 10000 gouttelettes par seconde.

A titre d'exemple, la source laser 64 est une source laser avec une longueur d'onde de 1064 nm.

En plus, de la source laser, le dispositif d'impression 50 comprend un système optique 68 qui permet un réglage de la focalisation selon un axe Z perpendiculaire à la surface de dépose 56. Avantageusement, le système optique 68 comprend une lentille qui permet de focaliser le faisceau laser 66 sur une zone impactée. De préférence, le système optique 68 comprend un miroir pour modifier la position de la zone impactée. Ainsi, le système optique 68 permet de modifier la zone impactée par le faisceau laser dans un plan d'impact référencé Pi sur la figure 3.

La source laser 64 et le système optique 68 ne sont pas plus décrits car ils sont connus de l'homme du métier et peuvent être identiques à ceux de l'art antérieur.

Le dispositif d'impression 50 comprend également au moins un support donneur 70 qui comprend selon un mode de réalisation une couche absorbante 72 à la longueur d'onde du faisceau laser 66 sur laquelle est apposé un film 74 d'au moins une encre biologique.

Pour la suite de la description, on entend par film que l'encre biologique occupe un volume avec une épaisseur (dimension selon une direction perpendiculaire au plan d'impact Pi) inférieure à 500 µ 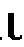 η).

Contrairement à un réservoir, le fait que l'encre biologique soit conditionnée sous la forme d'un film permet d'éviter les phénomènes de sédimentation.

La couche absorbante 72 est réalisée en un matériau adapté à la longueur d'onde du faisceau laser 66 pour transformer l'énergie lumineuse en un échauffement ponctuel de la couche absorbante 72.

De préférence, le support donneur 70 est positionné de manière à ce que le système optique focalise le faisceau laser au niveau de la couche absorbante 72.

Selon un mode de réalisation, la couche absorbante 72 est en or, en titane, ou autre en fonction de la longueur d'onde du faisceau laser 66.

Selon un autre mode de réalisation, le support donneur 70 ne comprend pas de couche absorbante 72. Dans ce cas, l'énergie du faisceau laser 66 est absorbée par l'encre.

De préférence, le support donneur 70 comprend une lame 76 en un matériau transparent à la longueur d'onde du faisceau laser 66 qui comprend sur une de ses faces un revêtement correspondant à la couche absorbante 72. La présence de la lame 76 confère une rigidité au support donneur 70 permettant de le manipuler et de conserver l'encre et/ou la couche absorbante 72 sensiblement plane dans le plan d'impact Pi.

Le film 74 d'encre biologique comprend une surface libre 78 qui est espacée de la couche absorbante 72 d'une distance E correspondant à l'épaisseur du film 74 et qui est espacée de la surface de dépose 56 d'une distance L. La surface libre 78 et la surface de dépose 56 se font face. Comme illustré sur la figure 3, le faisceau laser 66 est adapté pour produire une cavité 80 à l'interface entre la couche absorbante et le film 74 d'encre biologique qui génère une gouttelette 82 qui se détache de la surface libre 78 pour se déplacer vers la surface de dépose 56.

Pour la suite de la description, une direction verticale est parallèle à la force gravitationnelle G et le sens haut-bas correspond au sens de la force gravitationnelle G.

La direction du faisceau laser 66 et la direction du mouvement de la gouttelette sont parallèles à la direction verticale.

### Tir vers le haut :

Selon une caractéristique de l'invention, le faisceau laser 66 et donc le mouvement de la gouttelette 82 sont orientés à contresens par rapport à la force gravitationnelle G. Ainsi, la surface libre 78 du film 74 d'encre biologique est orientée vers le haut. Lors de son mouvement du film 74 d'encre biologique vers la surface de dépose 56, la gouttelette 82 se déplace vers le haut, selon le sens bas-haut.

Cette configuration procure les avantages suivants :
- Elle limite l'apparition des phénomènes de sédimentation, l'encre biologique étant sous la forme d'un film,
- Elle permet d'obtenir une épaisseur E pour le film 74 d'encre biologique sensiblement constante, l'influence de la force gravitationnelle G sur la forme de la surface libre 78 du film 74 étant limitée du fait que la surface libre 78 est orientée vers le haut,
- Elle permet d'utiliser une large gamme d'encre biologique lorsqu'une couche absorbante 72 indépendante du film 74 d'encre biologique est utilisée pour transformer l'énergie lumineuse en un échauffement ponctuel.

### Energie cinétique quasi nulle au moment du dépôt d'une gouttelette sur le substrat receveur:

La formation d'une gouttelette 82 à partir d'un film d'encre biologique va dépendre de nombreux paramètres, essentiellement des caractéristiques de l'encre biologique, des caractéristiques du faisceau laser et des conditions de réalisation. Sur les figures 4A à 4D, on a représenté l'évolution dans le temps de la déformation de la surface libre du film d'encre biologique, aboutissant ou non à la formation d'une gouttelette, pour différentes valeurs de l'énergie du faisceau laser 66, ce dernier ayant une énergie de 21 uJ pour la figure 4A, de 35 µϋ pour la figure 4B, de 40 uJ pour la figure 4C et 43 uJ pour la figure 4D.

Pour la même encre biologique et dans les mêmes conditions de réalisation, on note qu'il existe plusieurs régimes en fonction de l'énergie du faisceau laser.

Comme illustré sur la figure 4A, si l'énergie du faisceau laser est inférieure à un seuil inférieur, la gouttelette ne se détache pas du film 74 d'encre biologique. La hauteur maximale de la déformation 84 générée au niveau de la surface libre 78 du film 74 de l'encre étant inférieure à la distance L séparant le film 74 et la surface de dépose 56 aucun élément n'est imprimé. Selon l'exemple choisi, le seuil inférieur est compris entre 21 µϋ et 35 uJ. Comme illustré sur la figure 4D, si l'énergie du faisceau laser est supérieure à un seuil supérieur, la bulle de gaz 80 produite à l'intérieur du film éclate au niveau de la surface libre provoquant la projection incontrôlée de microgouttelettes. Selon l'exemple choisi, le seuil supérieur est compris entre 40 uJ et 43 µϋ.

Entre les seuils inférieur et supérieur, comme illustré sur les figures 4B et 4C, on est en présence d'un régime permettant la formation d'un jet. Si la distance L séparant le film 74 et la surface de dépose 56 est suffisante, ce régime permet la formation d'une gouttelette. De préférence, la distance L est de l'ordre de 1 à 2 mm pour permettre la formation d'une gouttelette et non d'un jet continu qui s'étire depuis le film jusqu'à la surface de dépose. Cette configuration limite les risques de contamination du tissu biologique réalisé par l'encre biologique.

Selon une autre caractéristique de l'invention, pour la même encre biologique et dans les mêmes conditions de réalisation, la distance L séparant le film 74 d'encre biologique et la surface de dépose 56 et/ou l'énergie du faisceau laser 66 sont ajustées de sorte que l'énergie cinétique de la gouttelette soit quasi nulle lorsque la gouttelette 82 touche la surface de dépose 56, comme illustré sur la figure 5D. Cette configuration limite le risque d'endommagement des éléments à imprimer qui sont des cellules.

Par quasi nulle, on entend que l'énergie cinétique est nulle ou très légèrement positive pour permettre à la gouttelette de se fixer sur la surface de dépose 56. Cette circonstance est rendue possible du fait que la gouttelette 82 se déplace à contresens par rapport la force gravitationnelle G.

De préférence, la distance L séparant le film 74 d'encre biologique et la surface de dépose 56 est fixe. Par conséquent, l'énergie du faisceau laser 66 est ajustée de sorte à ce que l'énergie cinétique de la gouttelette soit quasi nulle lorsque la gouttelette 82 touche la surface de dépose 56.

### Technique de calibration :

Comme indiqué précédemment, la formation de la gouttelette n'est pas uniquement liée à l'énergie du faisceau laser. Elle est également liée à la nature de l'encre biologique notamment de sa viscosité, de sa tension de surface et aux conditions de réalisation.

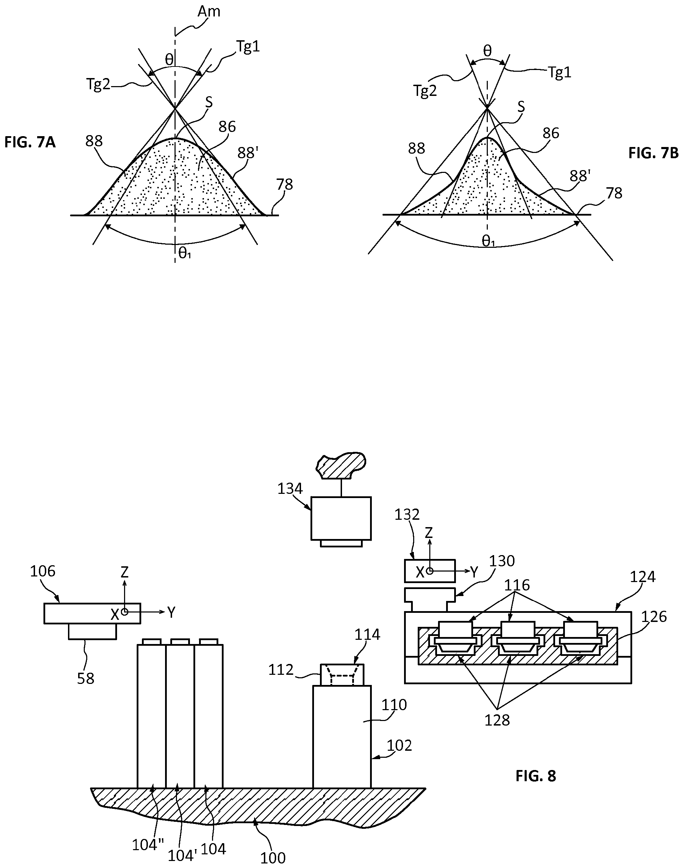

Sur les figures 5A à 5D, 7A à 7D, on a illustré un procédé de calibration permettant de déterminer l'énergie du faisceau laser pour obtenir un régime optimal concernant la formation et le dépôt des gouttelettes, notamment un régime qui conduit à un dépôt à vitesse nulle à une distance L donnée.

Sur les figures 5A à 5D, on a représenté certaines des étapes de formation d'une gouttelette 82 entre l'instant de l'impact du faisceau laser illustré sur la figure 5A et le dépôt de la gouttelette 82 sur la surface de dépose 56.

Selon une caractéristique de l'invention, le procédé de calibration permettant d'ajuster l'énergie du laser comprend les étapes consistant à mesurer un angle de pointe Θ d'une déformation 86 de la surface libre 78 du film 74 de l'encre biologique à un instant TI fixe après l'impact du faisceau laser 66 et à ajuster l'énergie du faisceau laser 66 en fonction de la valeur mesurée de l'angle de pointe Θ.

Comme illustré sur les figures 5B, 7A et 7B, la déformation 86 a une forme symétrique par rapport à un axe médian Am parallèle à la direction verticale. Cette déformation 86 comprend un sommet S centré par rapport à l'axe médian Am. Ce sommet S correspond à la zone de la déformation 86 la plus éloignée du reste de la surface libre 78 du film 74.

Dans un plan contenant l'axe médian Am, le sommet S est prolongé par un premier flanc 88 d'un côté de l'axe médian Am et par un second flanc 88' de l'autre côté de l'axe médian Am, les deux flancs 88, 88' étant symétriques par rapport à l'axe médian Am.

Chaque flanc 88, 88' comprend un point d'inflexion. Le premier flanc 88 comprend au niveau de son point d'inflexion une première tangente Tgl et le second flanc 88' comprend au niveau de son point d'inflexion une seconde tangente Tg2, les deux tangentes Tgl et Tg2 étant sécantes en un point de l'axe médian Am.

L'angle de pointe Θ correspond à l'angle formé par les tangentes Tgl et Tg2 et orienté vers le film 74 (soit vers le bas).

Pour obtenir la formation d'une gouttelette, l'angle de pointe Θ doit être inférieur ou égal à un premier seuil Θ1.

Ainsi, comme illustré sur la figure 7A, si l'angle de pointe Θ est supérieur au premier seuil Θ1, l'énergie du faisceau laser n'est pas suffisante pour générer une gouttelette. A contrario, comme illustré sur la figure 7B, si l'angle de pointe Θ est inférieur au premier seuil Θ1, l'énergie du faisceau laser est suffisante pour générer une gouttelette.

Pour obtenir une énergie cinétique quasi nulle au moment où la gouttelette formée atteint la surface de dépose 56 placée à une distance L de la surface libre 78 du film 74, l'angle de pointe Θ doit être supérieur ou égal à un second seuil Θ2.

De préférence, la valeur de l'angle de pointe Θ est déterminée grâce à une prise de vue à l'instant TI de la déformation 86. Selon un mode de réalisation, la prise de vue est réalisée grâce à une caméra dont l'axe de visée est perpendiculaire à la direction verticale.

L'instant TI est fonction de l'épaisseur du film et varie très peu d'une encre à l'autre. Avantageusement, l'instant TI est de l'ordre de 4 à 5 µ≤ à compter de l'impact du faisceau laser pour une épaisseur E de film de l'ordre de 40 à 50 µ 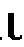 η. Cet instant TI correspond à la figure 5B.

Le premier seuil Θ1 est approximativement égal à 105°. Ainsi, si à l'instant TI l'angle de pointe Θ est inférieur ou égal à 105°, l'énergie du faisceau laser est suffisante pour générer une gouttelette 82.

Le second seuil Θ2 est fonction de la distance L entre la surface de dépose 56 et la surface libre 78 du film 74 de l'encre. Le second seuil Θ2 est inversement proportionnel à la distance L.

Le second seuil Θ2 est élevé et égal à approximativement 80° pour une distance L faible de l'ordre de 1 mm. Le choix d'une distance L relativement faible sera privilégié pour réduire les contraintes dans le jet et au moment du contact des gouttelettes avec la surface de dépose. Le second seuil Θ2 est faible et égal à approximativement 50° pour une distance L importante de l'ordre de 10 mm. Le choix d'une distance L relativement importante sera privilégié si on souhaite imprimer à longue distance par exemple si le support donneur 70 a des dimensions plus importantes que celles du puits au fond duquel est positionnée la surface de dépose 56.

Cette technique de calibration de l'énergie du faisceau laser permet d'optimiser la vitesse du jet en la réduisant pour limiter les risques d'endommagement des éléments contenus dans l'encre biologique notamment au moment du dépôt sur la surface de dépose 56.

### Epaisseur du film d'encre :

De préférence, l'encre biologique comprend une forte concentration en éléments à imprimer 62 afin d'obtenir un tissu biologique avec une forte concentration en cellules. Dans ce cas, comme illustré sur la figure 3, la gouttelette 82 comprend une fraction volumique en éléments à imprimer 62 élevée.

Pour les encres biologiques à forte concentration, l'épaisseur E du film 74 est de l'ordre de 40 à 60 µ 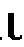 η).

Avantageusement, pour améliorer la précision du dépôt des éléments à imprimer, le film 74 d'encre biologique a une épaisseur E comprise entre 1,5D et 2D, D étant le diamètre des éléments à imprimer 62 qui ont une forme approximativement sphérique ou le diamètre d'une sphère dans laquelle un élément à imprimer 62 est inscrit.

Selon un mode de réalisation, le film 74 d'encre biologique a une épaisseur E supérieure ou égale à 20 µ 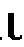 η pour les plus petits éléments à imprimer qui ont un diamètre de l'ordre de 10 à 15 µ 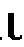 η. L'épaisseur E du film peut être de l'ordre de 400 µ 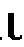 η lorsque les éléments à imprimer 62 sont des agrégats de cellules.

Généralement, l'épaisseur E du film est inférieure à 100 µ 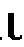 η lorsque les éléments à imprimer 62 sont des cellules unitaires.

De préférence, le film 74 se caractérise par un ratio (dimension de la surface libre 78)/(épaisseur du film 74) supérieur ou égal à 10, et avantageusement supérieur ou égal à 20. La dimension de la surface libre 78 correspond à la plus grande dimension de la surface libre 78 du film 74 prise dans un plan parallèle au plan d'impact Pi.

### Technique d'impression combinant une tête d'impression de type laser et une tête d'impression à buse :

Selon une autre caractéristique de l'invention, le procédé d'impression utilise au moins une tête d'impression de type laser pour au moins une première encre biologique et au moins une tête d'impression à buse pour au moins une deuxième encre biologique.

Cette combinaison permet d'augmenter le débit de fabrication.

Par tête d'impression à buse, on entend une tête d'impression qui comprend un orifice via lequel passe la deuxième encre biologique. Ainsi, une tête d'impression à buse peut être une tête d'impression de type jet d'encre, une tête d'impression par microvannes, une tête d'impression de type bioextrusion.

De préférence, chaque tête d'impression de type laser est identique à celle décrite sur la figure 3. Cependant, l'invention n'est pas limitée à cette tête d'impression de type laser. Ainsi, il est envisageable d'utiliser les têtes d'impression de type laser décrites sur les figures 1 et 2 ou d'autres.

Les têtes d'impression à buse ne sont pas plus décrites car elles sont de préférence identiques à celles de l'art antérieur.

Dans le cas d'un tissu biologique comprenant des cellules disjointes séparées par des matériaux extracellulaires, les matériaux extracellulaires sont déposés de préférence par la ou les tête(s) d'impression à buse et les cellules sont déposées de préférence par la ou les tête(s) d'impression de type laser.

Les matériaux extracellulaires étant moins sensibles aux effets de cisaillement, il est possible de les déposer avec une tête d'impression à buse. Les cartouches d'encre biologique destinées aux têtes d'impression à buse ayant un volume très nettement supérieur au volume d'encre (de l'ordre de 40µl) supporté par un support donneur 70 destiné à une tête d'impression de type laser, il est possible de déposer les matériaux de la matrice extracellulaire avec un débit important. Même si une tête d'impression à buse est capable de déposer les encres avec un débit important, chaque support donneur destiné à une tête d'impression de type laser supportant un très faible volume d'encre, il est nécessaire de les changer fréquemment ce qui tend à augmenter le temps de dépose par rapport à une tête d'impression à buse.

Selon une autre caractéristique, la ou les tête(s) d'impression de type laser et la ou les tête(s) d'impression à buse sont intégrées dans une même machine et se déplacent dans le même référentiel. Cette configuration permet de simplifier le positionnement relatif des différentes têtes d'impression, d'améliorer la précision de dépose et de garantir l'intégrité des éléments imprimés.

Dispositif d'impression comprenant une enceinte de stockage de supports donneurs :
Sur les figures 8 à 11, on a représenté un dispositif d'impression selon un mode de réalisation de l'invention.

Ce dispositif d'impression comprend un bâti 100 supportant une tête d'impression 102 de type laser et plusieurs têtes d'impression 104, 104', 104" de type jet d'encre. Ce bâti 100 comprend un repère X,Y,Z, l'axe Z étant orienté selon la direction verticale, le plan X,Y correspondant à un plan horizontal.

Les têtes d'impression 102, 104, 104', 104" sont fixes par rapport au bâti 100 et positionnées de manière à ce que les gouttelettes soient émises verticalement, vers le haut.

Les têtes d'impression 102, 104, 104', 104" sont décalées selon une première direction parallèle à l'axe Y. Selon un mode de réalisation, les têtes d'impression 104, 104', 104" de type jet d'encre sont accolées les unes contre les autres. La tête d'impression 102 de type laser est écartée des têtes d'impression 104, 104', 104" de type de jet.

Le dispositif d'impression comprend également un châssis mobile 106, un système de guidage et de déplacement du châssis mobile 106 par rapport au bâti 100 selon trois directions parallèles aux axes X,Y,Z et un système de commande permettant de contrôler les déplacements du châssis mobile 106. Le système de guidage et de déplacement et le système de contrôle sont choisis de manière à atteindre une précision micrométrique concernant les déplacements du châssis mobile 106 par rapport au bâti.

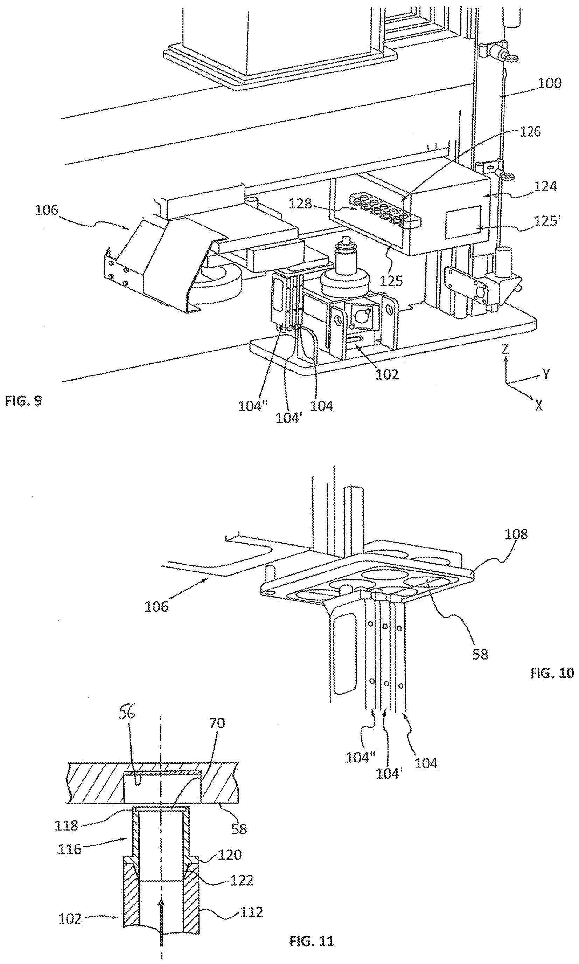

Comme illustré sur la figure 10, le châssis mobile 106 comprend un cadre 108 permettant de fixer de manière amovible au moins un substrat receveur 58. Lorsqu'il est solidarisé au châssis mobile, les déplacements du substrat receveur 58 sont contrôlés avec une précision micrométrique.

La tête d'impression 102 de type laser comprend un corps creux cylindrique 110, fixe par rapport au bâti, qui contient une partie d'un système optique et qui est surmonté d'une portion tubulaire 112 qui comprend une extrémité supérieure 114 placée qui débouche dans un plan horizontal. Ces éléments sont configurés de manière à ce qu'un faisceau laser guidé par le système optique balaie la section de l'extrémité supérieure 114.

Chaque support donneur 70 a la forme d'un disque positionné sur un socle 116. Selon un mode de réalisation illustré sur la figure 11, chaque socle 116 a la forme d'un tube qui comprend au niveau de son bord supérieur un décrochement 118 qui a un diamètre identique à celui d'un support donneur 70 et une hauteur suffisante pour le maintenir. Ainsi, ce décrochement 118 permet de positionner un support donneur 70 par rapport au socle qui le reçoit.

L'extrémité supérieure 114 et le socle 116 ont des formes qui coopèrent entre elles de manière à ce que le socle 116 soit immobilisé selon une position donnée par rapport à l'extrémité supérieure 114 et donc par rapport au repère X,Y,Z du bâti. Selon un mode de réalisation, le socle 116 comprend une collerette 120 extérieure qui prend appui contre l'extrémité supérieure 114 et permet de positionner le socle selon l'axe Z. En dessous de la collerette 120, le socle 116 comprend une surface tronconique 122 qui coopère avec une portion tronconique prévue à l'intérieur de la portion tubulaire 112. Ces formes permettent de centrer le socle 116 par rapport à la portion tubulaire 112 et de le positionner dans un plan XY. De préférence, il est possible d'utiliser des matériaux magnétiques pour améliorer le positionnement du socle 116 par rapport à la portion tubulaire 112.

Avantageusement, le dispositif d'impression comprend une enceinte 124 configurée pour stocker au moins un socle 116. Cette enceinte 124 comprend au moins une ouverture 125 permettant de faire entrer et sortir le ou les socles 116 stockés. Selon un mode de réalisation, cette enceinte 124 a une forme parallélépipédique.

De préférence, l'enceinte 124 a des dimensions adaptées pour pouvoir stocker plusieurs socles. Ainsi, le dispositif d'impression peut imprimer successivement plusieurs encres biologiques avec la même tête d'impression 102 de type laser.

Les socles 116 sont stockés sur une embase 126 qui comprend des logements 128, un logement pour chaque socle 116. L'embase 126 a une forme allongée et comprend sur sa longueur des encoches 128 en U. Selon une première variante illustrée sur la figure 8, la longueur de l'embase 126 est orientée selon l'axe Y.

Selon une seconde variante privilégiée, la longueur de l'embase 126 est orientée selon l'axe X et les encoches 128 sont ouvertes en direction des têtes d'impression.

Avantageusement, l'enceinte 124 comprend sur une première face orientée vers les têtes d'impression une première ouverture 125 permettant de faire sortir les socles 116 et sur une autre face une deuxième ouverture 125' permettant d'introduire les socles 116. Selon un mode de réalisation, l'enceinte 124 comprend un système de guidage pour positionner l'embase 126, par exemple un rail, l'embase 126 comprenant en partie inférieure une rainure dont la section coopère avec celle du rail. Ce rail débouche au niveau de la deuxième ouverture 125'. Il est de préférence orienté selon l'axe X.

L'enceinte 124 comprend des moyens de confinement pour conserver à l'intérieur de l'enceinte une atmosphère adaptée aux encres biologiques, notamment en température et/ou en hygrométrie. Ces moyens de confinement sont prévus notamment au niveau de chaque ouverture 125, 125'. Ils peuvent prendre la forme d'une barrière ou d'un rideau d'air. En complément de l'enceinte, le dispositif d'impression comprend une pince mobile 130 pour déplacer les socles entre l'enceinte 124 et la tête d'impression 102 de type laser. Selon une première variante, la pince mobile 130 est solidarisée à un chariot mobile 132, indépendant du châssis mobile 106, qui est configuré pour se déplacer selon les directions X, Y, Z.

Selon une autre variante, la pince mobile 130 est solidarisée au châssis mobile 106.

Selon un mode de réalisation, le dispositif d'impression comprend un appareil de prise de vue (non représenté) dont l'axe de visée est perpendiculaire à la direction verticale et orientée au niveau de la surface supérieure du support donneur. Cet appareil peut être utilisé pour calibrer l'énergie du faisceau laser de la tête d'impression 102 de type laser.

### Procédé de réalisation d'un tissu biologique par bio-impression :

La première étape dudit procédé consiste à générer une représentation numérique en trois dimensions du tissu biologique à imprimer.

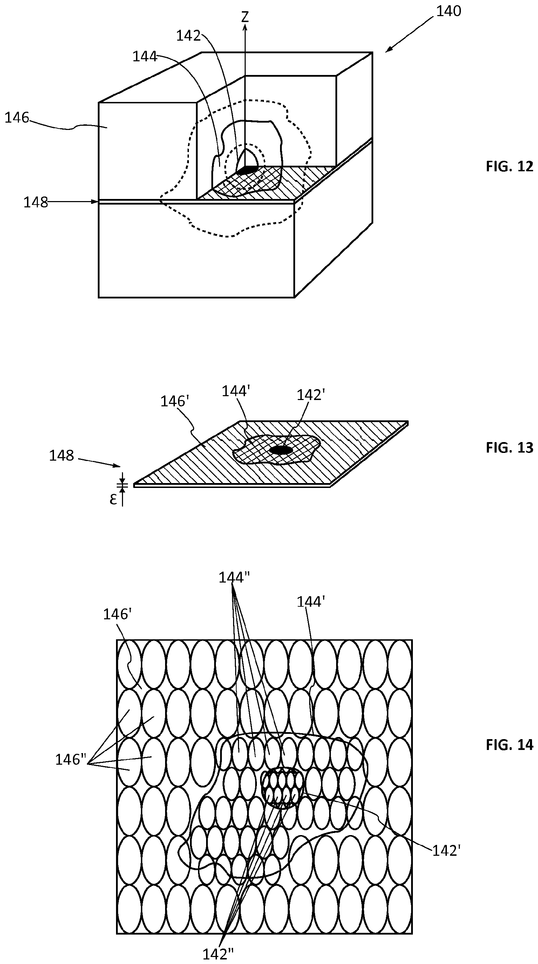

Sur la figure 12, on a représenté en 140 une partie d'une telle représentation sous la forme d'un cube qui comprend une première zone volumique 142 placée à l'intérieur d'une deuxième zone volumique 144 elle-même disposée dans une troisième zone volumique 146. Pour les besoins de la description, la représentation 140 est grandement simplifiée.

Chaque zone volumique 142, 144, 146 est colorée ou texturée différemment, chaque couleur ou texture correspondant à un ensemble de caractéristiques parmi les caractéristiques suivantes (non limitées) matériau, moyen de fabrication, trajectoire,...

De préférence, chaque couleur ou texture correspond à une encre biologique.

Toutes les zones volumiques 142, 144 et 146 sont fermées. Avantageusement, la représentation comprend une pluralité de petits volumes élémentaires qui ont des couleurs ou textures différentes en fonction de la zone volumique à laquelle ils appartiennent. Selon un mode de réalisation, la représentation 140 est issue d'un fichier informatique de type PLY.

La deuxième étape du procédé consiste à trancher la représentation 140 en une succession de couches superposées selon un axe Z. Sur la figure 13, on a isolé une couche 148 de la représentation 140.

Lors du tranchage de la représentation 140, au droit d'un changement de zone volumique, chaque couche comprend une arête qui correspond à un changement de zone.

Comme illustré sur la figure 13, la couche 148 comprend une première zone 142' qui correspond à la première zone volumique 142, une deuxième zone 144' qui correspond à la deuxième zone volumique 144 et une troisième zone 146' qui correspond à la troisième zone volumique 146. Pour chaque couche, les zones 142', 144', 146' sont colorées ou texturées en fonction de la couleur ou texture des zones volumiques 142, 144, 146.

Chaque couche a une épaisseur ε qui est déterminée en fonction de la hauteur des gouttelettes imprimées.

Si la couche ne comprend qu'une seule matière à imprimer, la couche a une épaisseur sensiblement égale à la hauteur d'une gouttelette.

Lorsque la couche comprend plusieurs matières à imprimer, selon une première variante, la couche a une épaisseur égale au plus petit commun multiple des hauteurs des gouttelettes associées à chaque matière. Cette variante a pour avantage de minimiser le décalage dans toute la hauteur de l'objet à imprimer et d'aboutir à une impression rapide.

Selon une deuxième variante, la couche a une épaisseur égale au plus grand commun diviseur des hauteurs des gouttelettes associées à chaque matière. Cette variante a pour avantage d'augmenter la résolution et le nombre de couches.

A titre d'exemple, si la première matière est imprimée par bio-impression par laser, les gouttelettes imprimées ont une hauteur de l'ordre de 10 µ 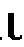 η. Si la deuxième matière est imprimée par bio-impression par microvannes, les gouttelettes imprimées ont une hauteur de l'ordre de 100 µ 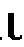 η. Selon la première variante, les couches ont une épaisseur de l'ordre de 100 µ 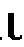 η. Selon la deuxième variante, les couches ont une épaisseur de l'ordre de 10 µ 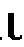 η.

De préférence, chaque couche comprend une pluralité de petits polygones élémentaires par exemple triangulaires qui ont des couleurs différentes en fonction de la zone à laquelle ils appartiennent.

Ainsi, l'objet à imprimer correspond à un ensemble de couches qui comprennent chacune un ensemble de polygones qui ont chacun une couleur ou une texture associée.

Une troisième étape du procédé consiste à déterminer pour chaque couche la position des gouttelettes à imprimer de chaque encre biologique en fonction des zones 142', 144', 146' colorées ou texturées et du volume prévu de chacune des gouttelettes. A cet effet, chaque zone 142', 144', 146' de chaque couche est remplie par des ellipses 142", 144", 146" dont les dimensions sont fonction des dimensions des gouttelettes de l'encre biologique destinée à être imprimée dans ladite zone, comme illustré sur la figure 14.

Pour chaque zone, les ellipses ont les mêmes dimensions. Toutes les ellipses ont des axes focaux parallèles.

La forme elliptique permet de pouvoir adapter les distances entre les gouttelettes selon deux directions (une première direction parallèle aux axes focaux et une seconde direction perpendiculaire à la première).

Le centre de chaque ellipse correspond à la position du centre d'une gouttelette.

Le positionnement des ellipses se fait zone par zone, par ordre décroissant de grosseurs, Ainsi, les plus grosses ellipses disposées dans la zone 146' sont positionnées en premier et les plus petites ellipses disposées dans la zone 142' sont positionnées en dernier.

De préférence, au niveau d'un changement de zone, l'optimisation du positionnement se fait selon deux critères :
- ratio maximal de polygones élémentaires ayant la bonne couleur ou texture au sein d'une ellipse, de l'ordre de 75% par exemple,
- ratio minimal de polygones élémentaires ayant la mauvaise couleur ou texture au sein d'une ellipse, de l'ordre de 5% par exemple.

Les chevauchements entre ellipses peuvent être tolérés.

Une quatrième étape du procédé consiste à synchroniser le déplacement de la surface de dépose 56 sur laquelle les gouttelettes d'encre biologique sont imprimées et les différentes têtes d'impression.

Pour une bio-impression par laser, la zone de focalisation du laser est le centre de chaque ellipse imprimée par laser, et chaque ellipse fait l'objet d'un puise laser. Dans ce cas, la surface de dépose est fixe, c'est le laser qui balaie toute la surface de dépose. Pour une surface de dépose plus grande que le support donneur, Il est possible de déplacer également le substrat (sur lequel sont rapportées les surfaces de dépose) de manière synchronisée avec le balayage du laser.

Pour une bio-impression à buse, le centre de chaque ellipse correspond au point d'impact supposé d'une gouttelette sur la surface de dépose 56. Dans ce cas, la buse d'impression est fixe, c'est le substrat qui se déplace. Toutefois, la buse d'impression pourrait être mobile.

### Applications :

La bio-impression selon l'invention peut être utilisée pour produire :
- Des tissus implantables pour la médecine régénératrice,
- Des tissus individualisés, réalisés à partir des cellules du patient, permettant de sélectionner in vitro les traitements et de développer des solutions thérapeutiques personnalisées,
- Des modèles prédictifs reproduisant la physiologie de tissus humains sains ou de tissus affectés par une pathologie pour tester de manière prédictive l'efficacité ou la toxicité des molécules, d'ingrédients et des candidats médicaments.

A titre d'exemple et de manière non limitative, le tissu biologique est un tissu osseux.

## Revendications

1. Procédé d'impression d'au moins une encre biologique, ledit procédé utilisant au moins une tête d'impression (102) de type laser pour déposer au moins une gouttelette (82) d'au moins une encre biologique sur une surface de dépose (56) d'un substrat receveur (58), **caractérisé en ce que** le procédé d'impression utilise au moins une tête d'impression (104, 104', 104") à buse pour déposer au moins une gouttelette d'au moins une encre biologique sur une surface de dépose (56) du même substrat receveur (58) que la tête d'impression (102) de type laser.

2. Procédé d'impression selon la revendication 1, ledit procédé étant utilisé pour obtenir un tissu biologique comprenant des cellules et une matrice extracellulaire, **caractérisé en ce que** les matériaux constituant la matrice extracellulaire sont déposés par la ou les tête(s) d'impression (104, 104', 104") à buse et **en ce que** les cellules sont déposées par la ou les tête(s) d'impression (102) de type laser.

3. Procédé de réalisation d'un tissu biologique utilisant le procédé d'impression selon la revendication 1 ou 2, **caractérisé en ce qu'**il comprend les étapes visant à :
- Générer une représentation (140) numérique en trois dimensions du tissu biologique à réaliser, ladite représentation comprenant plusieurs zones volumiques (142, 144, 146) colorées ou texturées, chaque couleur ou texture étant associée à une encre biologique,
- Trancher la représentation (140) en une succession de couches (148) superposées, chaque couche (148) comprenant des zones (142', 144', 146') colorées ou texturées qui correspondent aux zones volumiques (142, 144, 146) de la représentation (140),
- Pour chaque couche, déterminer la position des gouttelettes à imprimer de chaque encre biologique en fonction des zones (142', 144', 146') colorées ou texturées et du volume prévu de chacune des gouttelettes,
- Imprimer les différentes gouttelettes.

4. Procédé de réalisation d'un tissu biologique selon la revendication 3, **caractérisé en ce qu'**un procédé d'impression selon la revendication 1 ou 2 est utilisé pour imprimer les différentes gouttelettes.

5. Procédé de réalisation d'un tissu biologique selon la revendication 3 ou 4, **caractérisé en ce que** les couches (148) ont une épaisseur fonction des dimensions des gouttelettes.

6. Procédé de réalisation d'un tissu biologique selon l'une des revendications 3 à 5, **caractérisé en ce que** chaque couche comprend un ensemble de petits polygones élémentaires qui ont des couleurs ou des textures différentes en fonction de la zone à laquelle ils appartiennent.

7. Procédé de réalisation d'un tissu biologique selon l'une des revendications 3 à 6, **caractérisé en ce que** pour déterminer la position de chaque gouttelette, chaque zone (142', 144', 146') est remplie par des ellipses (142", 144", 146") identiques qui ont des dimensions fonction des dimensions des gouttelettes de l'encre biologique destinée à être imprimée dans ladite zone, le centre de chaque ellipse correspondant à la position du centre d'une gouttelette.

8. Procédé de réalisation d'un tissu biologique selon la revendication 7, **caractérisé en ce que** le positionnement des ellipses est réalisé zone par zone, par ordre décroissant de grosseurs.

9. Dispositif d'impression comprenant au moins un substrat receveur (58) avec une surface de dépose (56) et au moins une tête d'impression (102) de type laser qui comprend :
- au moins une source laser (64) pulsée configurée pour émettre un faisceau laser (66),
- un système optique (68) pour focaliser et orienter ledit faisceau laser (66),
- au moins un support donneur (70) qui comprend au moins une encre biologique, **caractérisé en ce que** le dispositif d'impression comprend au moins une tête d'impression (104, 104', 104") à buse pour imprimer au moins une encre biologique sur le même substrat receveur (58) que la ou les tête(s) d'impression (102) de type laser.

10. Dispositif d'impression selon la revendication 9, **caractérisé en ce que** le dispositif d'impression comprend une enceinte (124) configurée pour stocker au moins un socle (116) supportant un support donneur (70), ladite enceinte (124) étant équipée de moyens de confinement permettant de conserver à l'intérieur une atmosphère adaptée aux encres biologiques.

11. Dispositif d'impression selon la revendication 10, **caractérisé en ce que** l'enceinte (124) a des dimensions adaptées pour stocker plusieurs socles (116) et **en ce que** le dispositif d'impression comprend au moins une embase (126) qui comprend des logements (128), un logement pour chaque socle (116), ladite embase étant configurée pour être stockée à l'intérieur de ladite enceinte (124).

12. Dispositif d'impression selon la revendication 11, **caractérisé en ce que** l'embase (126) comprend un système de guidage pour la positionner dans l'enceinte (124).

13. Dispositif d'impression selon l'une des revendication 9 à 12, **caractérisé en ce que** l'enceinte (124) comprend sur une première face orientée vers les têtes d'impression une première ouverture (125) permettant de faire sortir les socles (116) et sur une autre face une deuxième ouverture (125') permettant d'introduire les socles (116).

14. Dispositif d'impression selon l'une des revendications 9 à 13, **caractérisé en ce que** le dispositif d'impression comprend une pince mobile (130) pour déplacer les socles entre l'enceinte (124) et la tête d'impression de type laser (102).

15. Dispositif d'impression selon l'une des revendications 9 à 14, **caractérisé en ce que** le dispositif d'impression comprend un châssis mobile (106) supportant au moins un substrat récepteur (58), un système de guidage et de déplacement du châssis mobile (106) par rapport à un bâti (100) selon trois directions et un système de commande permettant de contrôler les déplacements du châssis mobile (106), ledit système de guidage et de déplacement et ledit système de contrôle ayant une précision micrométrique.

16. Dispositif d'impression selon les revendications 14 et 15, **caractérisé en ce que** la pince mobile (130) est solidarisée au châssis mobile (106).

## Patentansprüche

1. Druckverfahren mit mindestens einer biologischen Drucksubstanz, in dem mindestens ein Laser-Druckkopf (102) zum Auftragen von mindestens einem Tröpfchen (82) mindestens einer biologischen Drucksubstanz auf einer Auftragsfläche (56) eines Aufnahmesubstrats (58) benutzt wird, **dadurch gekennzeichnet, dass** in dem Druckverfahren mindestens ein Düsen-Druckkopf (104, 104', 104") zum Auftragen von mindestens einem Tröpfchen mindestens einer biologischen Drucksubstanz auf einer Auftragsfläche (56) des gleichen Aufnahmesubstrats (58) benutzt wird, das auch vom Laser-Druckkopf (102) verwendet wird.

2. Druckverfahren nach Anspruch 1, wobei besagtes Verfahren zur Herstellung eines biologischen Gewebes bestehend aus Zellen und einer extrazellularen Matrix benutzt wird, **dadurch gekennzeichnet, dass** die Materialien, welche die extrazellulare Matrix bilden, durch den oder die Düsen-Druckköpfe (104, 104', 104'') aufgetragen werden und dadurch, dass die Zellen durch den oder die Laser-Druckköpfe (102) aufgetragen werden.

3. Verfahren zur Herstellung eines biologischen Gewebes, bei dem das Druckverfahren nach Anspruch 1 oder 2 benutzt wird, **dadurch gekennzeichnet, dass** es Schritte umfasst, die darauf abzielen:
- eine dreidimensionale digitale Darstellung (140) des herzustellenden biologischen Gewebes zu generieren, wobei besagte Darstellung mehrere farbige oder texturierte Volumenbereiche (142, 144, 146) enthält, wobei jede Farbe oder Textur mit einer biologischen Drucksubstanz verknüpft ist,
- die Darstellung (140) in eine Abfolge übereinander gelagerter Schichten (148) zu schneiden, wobei jede Schicht (148) farbige oder texturierte Bereiche (142', 144', 146') enthält, die den Volumenbereichen (142, 144, 146) der Darstellung (140) entsprechen,
- für jede Schicht die Position der zu druckenden Tröpfchen jeder biologischen Drucksubstanz im Hinblick auf die farbigen oder texturierten Bereiche (142', 144', 146') und das für jedes Tröpfchen vorgesehene Volumen zu bestimmen,
- die verschiedenen Tröpfchen zu drucken.

4. Verfahren zur Herstellung eines biologischen Gewebes nach Anspruch 3, **dadurch gekennzeichnet, dass** zum Drucken der verschiedenen Tröpfchen ein Druckverfahren nach Anspruch 1 oder 2 benutzt wird.

5. Verfahren zur Herstellung eines biologischen Gewebes nach Anspruch 3 oder 4, **dadurch gekennzeichnet, dass** die Schichten (148) eine Dicke aufweisen, die von der Größe der Tröpfchen abhängt.

6. Verfahren zur Herstellung eines biologischen Gewebes nach einem der Ansprüche 3 bis 5, **dadurch gekennzeichnet, dass** jede Schicht eine Reihe kleiner elementarer Vielecke mit unterschiedlichen Farben oder Texturen aufweisen, je nachdem, zu welchem Bereich sie gehören.

7. Verfahren zur Herstellung eines biologischen Gewebes nach einem der Ansprüche 3 bis 6, **dadurch gekennzeichnet, dass** zur Bestimmung der Position jedes Tröpfchens jeder Bereich (142', 144', 146') mit identischen Ellipsen (142", 144", 146") gefüllt ist, deren Größe von der Tröpfchengröße der biologischen Substanz abhängt, die in besagtem Bereich gedruckt werden soll, wobei die Mitte jeder Ellipse und die Position des Mittelpunkts des Tröpfchens übereinstimmen.

8. Verfahren zur Herstellung eines biologischen Gewebes nach Anspruch 7, **dadurch gekennzeichnet, dass** die Ellipsen Bereich für Bereich in abnehmender Reihenfolge der Dicke positioniert werden.

9. Druckvorrichtung mit mindestens einem Aufnahmesubstrat (58) mit einer Auftragsfläche (56) und mindestens einem Laser-Druckkopf (102), die folgendes umfasst:
- mindestens eine Impulslaserquelle (64), die so gestaltet ist, dass sie einen Laserstrahl (66) emittiert,
- ein optisches System (68) zum Fokussieren und Ausrichten des besagten Laserstrahls (66),
- mindestens ein Donorelement (70) mit mindestens einer biologischen Drucksubstanz, **dadurch gekennzeichnet, dass** das Druckverfahren mindestens einen Düsen-Druckkopf (104, 104', 104") aufweist, um mindestens eine biologische Drucksubstanz auf dem gleichen Aufnahmesubstrat (58) zu drucken, das auch von dem oder den Laser-Druckköpfen (102) verwendet wird.

10. Druckverfahren nach Anspruch 9, **dadurch gekennzeichnet, dass** die Druckvorrichtung ein Gehäuse (124) umfasst, das so gestaltet ist, dass mindestens ein Träger (116) mit Donorelement (70) gelagert werden kann, wobei besagtes Gehäuse (124) mit Einschlussmitteln ausgestattet ist, die die Beibehaltung einer für die biologischen Drucksubstanzen geeigneten Atmosphäre im Innern ermöglicht.

11. Druckverfahren nach Anspruch 10, **dadurch gekennzeichnet, dass** das Gehäuse (124) so bemessen ist, dass es sich zur Einlagerung mehrerer Träger (116) eignet, und dadurch, dass die Druckvorrichtung mindestens einen Sockel (126) mit Aufnahmen (128) für jeweils einen Träger (116) umfasst, wobei der Sockel so gestaltet ist, dass er im Innern des Gehäuses (124) gelagert werden kann.

12. Druckvorrichtung nach Anspruch 11, **dadurch gekennzeichnet, dass** der Sockel (126) ein Führungssystem aufweist, um ihn im Gehäuse (124) zu positionieren.

13. Druckvorrichtung nach einem der Ansprüche 9 bis 12, **dadurch gekennzeichnet, dass** das Gehäuse (124) an einer ersten, zu den Druckköpfen ausgerichteten Seite eine erste Öffnung (125) aufweist, aus welcher die Träger (116) entnommen, und auf einer anderen Seite eine zweite Öffnung (125') aufweist, durch welche die Träger (116) eingesetzt werden können.

14. Druckvorrichtung nach einem der Ansprüche 9 bis 13, **dadurch gekennzeichnet, dass** die Druckvorrichtung einen beweglichen Greifer (130) umfasst, um die Sockel zwischen dem Gehäuse (124) und dem Laser-Druckknopf (102) zu verstellen.

15. Druckvorrichtung nach einem der Ansprüche 9 bis 14, **dadurch gekennzeichnet, dass** die Druckvorrichtung einen beweglichen Rahmen (106) umfasst, der mindestens ein Aufnahmesubstrat (58), ein Führungs- und Verschiebungssystem des beweglichen Rahmens (106) in Bezug auf ein Gestell (100) in drei Richtungen, und ein Bediensystem zur Kontrolle der Bewegungen des beweglichen Rahmens (106) trägt, wobei das Führungs- und das Verschiebungssystem und besagtes Kontrollsystem eine mikrometrische Genauigkeit aufweisen.

16. Druckvorrichtung nach den Ansprüchen 14 und 15, **dadurch gekennzeichnet, dass** der bewegliche Greifer (130) fest mit dem beweglichen Rahmen (106) verbunden ist.

## Claims

1. A method for printing with at least one bio-ink. Said method uses at least one laser print head (102) to deposit at least one droplet (82) of at least one bio-ink onto a depositing surface (56) of a receiving substrate (58). Said method is **characterized in that** the printing method uses at least one nozzle print head (104, 104', 104") to deposit at least one droplet of at least one bio-ink onto a depositing surface (56) of the same receiving substrate (58) as the laser print head (102).

2. A method for printing in line with Claim 1, where the said method is used to obtain a biological tissue comprising cells and an extracellular matrix and is **characterized in that** the constituents of the extracellular matrix are deposited by the nozzle print head(s) (104, 104', 104") and that the cells are deposited by the laser-type print head(s) (102).

3. A method for producing biological tissue using a printing method according to Claims 1 or 2, **characterized in that** it comprises steps aimed at:
- Generating a three-dimensional digital representation (140) of the biological tissue to be created, with the said representation comprising several coloured or textured volume areas (142, 144, 146), with each colour or texture being associated to a given bio-ink;
- Slicing the representation (140) into a succession of superimposed layers (148), with each layer (148) comprising coloured or textured zones (142', 144', 146') corresponding to the volumetric areas (142, 144, 146) of the representation (140);
- Determining, for each layer, the position of the droplet to be printed for each bio-ink, depending on the coloured or textured zones (142', 144', 146') and the rated volume of each droplet;
- Printing the various droplets.

4. Method for producing a biological tissue according to Claim 3, **characterized in that** a printing method according to Claim 1 or 2 is used to print different droplets.

5. Method for producing a biological tissue according to Claim 3 or 4, **characterized in that** the thickness of the layers (148) is determined by the size of the droplets.

6. Method for producing a biological tissue according to any of Claims 3 to 5, **characterized in that** each layer comprises a set of small and basic polygons of varying colours or textures depending on zone to which they belong.

7. Method for producing a biological tissue according to any of Claims 3 to 6, **characterized in that** for the position of each droplet to be determined, each zone (142', 144', 146') is filled by identical ellipses (142", 144", 146") where the dimensions of these ellipses are determined by the amounts of the biological ink to be printed in the said zones, and the centre of each zone corresponds to the central position of the droplet.

8. Method for producing a biological tissue according to Claim 7, **characterized in that** the locations of the ellipses are determined zone by zone, from the largest to the smallest.

9. Printing device comprising at least one receiving substrate (58) with a depositing surface (56) and at least one laser-type print head (102) with:
- at least one pulsed laser source (64) configured to emit a laser beam (66),
- an optical system (68) to focus and orientate the laser beam (66),
- at least one donor support (70) comprising at least one bio-ink and **characterized in that** the printing device comprises at least one nozzle print head (104, 104', 104") that prints at least one biological ink on the same receiving substrate (58) as the laser-type print head(s) (102).

10. Printing device according to Claim 9, **characterized in that** the printing device comprises an enclosed area (124) configured to store at least one pedestal base (116) carrying the donor support (70), with the said enclosure (124) being equipped with means of confinement for keeping the bio-ink in an appropriate atmosphere.

11. Printing device according to Claim 10, **characterized in that** the enclosure (124) has suitable dimensions for storing several pedestal bases (116) and **in that** the printing device comprises at least one hub (126) with housings (128), one housing for each pedestal base (116), and the said hub being configured to be stored within the said enclosure (124).

12. Printing device according to Claim 11, **characterized in that** the hub (126) comprises a guiding system to position the enclosure (124).

13. Printing device according to Claims 9 to 12, **characterized in that** the enclosure (124) comprises one opening on one face on the side of the print heads (125) that allows the pedestal base to be removed (116) and another opening (125') on the other face, that allows the introduction of the pedestal bases (116).

14. Printing device according to Claims 9 to 13, **characterized in that** the printing mechanism comprises mobile clamps (130) for moving the pedestal bases between the enclosure (124) and the laser-type print head (102).

15. Printing device according to Claims 9 to 14, **characterized in that** the printing mechanism comprises a mobile frame (106) carrying at least one receiving substrate (58), a system for guiding and moving the frame (106) that moves in relation to a follower (100) and in three possible directions, together with a command system to control the movements of the mobile frame (106), with the said mobile frame guiding, movement and command systems that has a micrometre accuracy.

16. Printing device according to Claims 14 and 15, **characterized in that** the mobile clamp (130) is attached to the mobile frame (106).
